# NEW EUROPEAN PATENT SPECIFICATION

(11) **EP 3 288 512 B2**
(45) Date of publication and mention of the opposition decision: **23.07.2025**
(45) Mention of the grant of the patent: 19.01.2022
(21) Application number: 16725197.4
(22) Date of filing: 27.04.2016
(51) Int. Cl.: A61F 13/02, A61F 13/00

(54) **WOUND DRESSING**
WUNDAUFLAGE
PANSEMENT

(30) Priority: 27.04.2015 GB 201507134
(43) Date of publication of application: 07.03.2018
(73) Proprietor: Medtrade Products Limited, Crewe, Cheshire CW1 6GL (GB)
(72) Inventor: HOGGARTH, Andrew, Crewe Cheshire CW2 6TA (GB); BUGEDO, Ander, Winsford Cheshire CW7 2LF (GB); HARDY, Craig, St Dogmaels Cardigan SA43 3BH (GB)
(74) Representative: Wilson Gunn
(86) International application number: PCT/GB2016/051179
(87) International publication number: WO 2016/174419

(56) References cited:
- WO-A1-2004/058214
- WO-A1-2010/117636
- WO-A1-2014/140608
- WO-A2-2010/129299
- CA-A1- 2 268 344
- CN-A- 103 655 046
- GB-A- 2 464 970
- GB-A- 983 576
- US-A- 3 122 140
- US-A- 3 122 141
- US-A- 5 437 621
- US-A1- 2004 209 067
- US-A1- 2010 247 844
- US-A1- 2014 058 310

## Description

The present invention relates to a wound dressing, and also to methods of making the wound dressing.

Topical wound dressings for use in the treatment of wounds or other openings at a physiological target site on a human or animal body which are exuding blood and/or other bodily fluids have been known for some time. The materials used to make the wound dressings act to absorb the blood and/or other bodily fluids, and also stem the flow of them from the body. Materials for wound dressings are described in, for example, WO2010031995 and PCT/GB2015/050816 to MedTrade Products Limited, and are commercially available.

The management of exudate is of course essential and critical during wound care and surgical procedures. The aim of managing the exudate is essentially to provide a moist wound environment at the wound bed to minimise the risk of maceration, which in turn may reduce the negative impact upon the human or animal body and also shorten the length of time the patient will take to recover.

Wound dressings often comprise at least a quantity of an absorbent material. The purpose of the absorbent material is to absorb wound exudate from the wound, thereby drawing it away from the wound bed. This avoids the wound bed being overly wet which, as noted above, can be detrimental to the healing process. A further development in wound management is the use of all over adhesive systems, particularly silicones, whereby the adhesive covers the surface of the wound contact layer. This has its advantages in that it allows the dressing to maintain an intimate contact with the wound, adhering only to drier areas and not wet areas. Also, where there are drier areas within the wound area, adhesion of the dressing increases its adhesive contact and as such increases its ability to stay in place, compared to adhesives solely within the dressing border area. Silicone adhesive systems are used as they reduce the likelihood of skin stripping, both in the wound area and the peri-wound area.

Within these dressings, there is often a fluid wicking layer on the surface of the adhesive wound contact layer distal to the wound bed, to which a highly absorbent layer is bonded. This absorbent layer often comprises superabsorbent materials, which not only have a high fluid absorbency potential but also a high fluid retention ability, reducing the risk of wound and peri-wound maceration. On manufacturing of the final product construction, the addition of the adhesive wound contact layer over the wicking layer, whether directly applied or applied using a carrier layer, can significantly reduce the hydrophobicity of the wicking layer, such that a droplet of fluid (solution A, water, saline, simulated wound fluid or exudate) can take in excess of 60 seconds to absorb into the wicking layer and through to the superabsorbent layer. This is not ideal as it may result in excess fluid between the adhesive wound contact layer and the wound bed, trapping exudate which may impede healing in that it may slow down or prevent cell proliferation, may interfere with growth factor availability or may contain elevated levels of inflammatory mediators and activated MMPs.

Further benefits to this invention are that it allows hydrophobic materials to be used as the wicking layer, potentially reducing the cost of the final dressing or being able to use more dense materials that offer support and comfort when wearing the dressing. In such situations, there may be no adhesive wound contact layer, such that the wicking layer is the wound contact layer.

As a simplistic overview, wound dressings manage wound exudate by three properties, fluid absorbency, fluid retention and moisture vapour transmission rates. Fluid absorbency is important to wick excess fluid from the wound bed, minimising the risk of maceration. Fluid retention post fluid absorbency is important in that it minimises the ability for fluid to migrate out of the dressing back into the wound bed or peri-wound area, again minimising the risk of wound and skin maceration. To try and minimise the dressing reaching maximum absorbency and to prolong usability of the dressing, moisture vapour transmission rates in the outer layer of the dressing are used, in essence allowing moisture to transfer to the atmosphere helping manage the fluid through the dressing. The combination of absorbency and moisture vapour transmission rates is termed total fluid handling.

For wound dressings whereby the use of an all over adhesive wound contact layer impedes the rate of fluid uptake, potentially increasing the risk of wound maceration and delayed healing, there is a need to improve this rate of fluid uptake.

US 2010/247844 describes a laminate web having a nonwoven web in facing relationship with a polymer film, the laminate web having a first side comprising the polymer film and a plurality of discrete tufts including fibers integral with and extending from the nonwoven web. GB 2464970 describes a wound dressing comprises fibrous plugs within a foam sheet. US 2014/058310 describes a wound dressing essentially consisting of an absorbent nonwoven compress formed from a mixture of bicomponent thermal bonding non-absorbent fibers, of core-shell type, the core being made of polyester and the shell being made of polyethylene; of bicomponent superabsorbent fibers of core-shell type with a core made of polyacrylonitrile and a shell made of polyacrylate; and a net fabric made from a hydrocolloid elastomer mass partially covering the face of the compress intended to come into contact with the wound. CA 2268344 describes a non-woven absorbent composite for use mainly as disposable sanitary or medical article, having an upper layer of a suitable open fibrous structure and a lower layer of a relatively dense fibrous structure.

There therefore remains a need for a composition suitable for use as or in a wound dressing that can address the aforementioned problems. The present invention has been arrived at with the foregoing in mind.

In order to address this problem by increasing the rate of fluid uptake whilst not compromising the total fluid handling, wound dressings comprising an absorbent fibre material in the form of a textile in combination with a fluid wicking absorbent layer whereby the absorbent fibre layer is punched into the fluid wicking layer have been prepared. In such wound dressings, the action of punching the absorbent fibre material into the body of the absorbent wicking layer, significantly affects the rate of fluid uptake and reduces the time taken for fluid to be absorbed. It also maintains and has no detrimental effect to the total fluid handling characteristics.

According to the present invention, there is provided a wound dressing composition comprising a first layer of a wicking material and a second layer of an absorbent fibre material, wherein the wicking material comprises a foam, and the absorbent fibre material is a nonwoven superabsorbent material, whereby the absorbent fibre material is punched into and/or through the layer of wicking material;
wherein at least a part of the absorbent material is exposed on a wound facing surface of the wicking layer; and
wherein prior to punching the absorbent material through the wicking material, the wicking material and the absorbent fibre material are bonded together by an adhesive to form bonded layers.

By 'punched' is meant herein that a plurality of needles creates a plurality of small holes in the layer of wicking material, such that the fibres of the absorbent material are able to pass or penetrate through the layer of wicking material. It is this modification of the layer of wicking material which enables the speed of fluid absorption by the wound dressing composition to be increased.

As a result of the punching process, at least a part of the absorbent material is exposed on a wound facing surface of the wicking layer.

The term 'absorbent material' is used herein to refer to a physiologically acceptable material that is capable of absorbing fluid, such as wound exudate, and which is capable of absorbing fluid to greater than about 500% by weight of the absorbent material, and with a fluid retention of greater than about 40%. The absorbent material referred to herein is a nonwoven superabsorbent material.

The term 'superabsorbent material' is used herein to refer to a hydrophilic material that is water-swellable, but not water soluble, and which is capable of absorbing fluid to greater than about 2000% by weight of the superabsorbent material, preferably greater than about 2500%, with a fluid retention of greater than about 85%, preferably greater than about 90%.

According to one embodiment, a further adhesive wound contact layer may be attached to the wound contact side of the wound dressing, *i.e.* on the side of the wicking layer proximal to the wound or physiological target site.

As noted herein, the wicking material will absorb wound fluid and thus draw it away from the wound bed. This has the advantageous effect of reducing the volume of fluid at the wound bed, thus avoiding a wound bed that is overly wet by creating a moisture level that is more conducive to wound healing. It is preferable to draw the wound fluid away from the wicking material, as over saturation of the wicking material results in an overly saturated wound bed.

The wicking layer can be a hydrophilic absorbent layer, or can be a hydrophobic low absorbent layer. It functions to wick fluid through to the superabsorbent layer and acts as a semi-barrier to the absorbent material entering the wound and also reduces moisture loss back into the wound.

In the present invention, the wicking material and the absorbent material can act in synergy, whereby the wicking material initially readily absorbs fluid from the wound site and the absorbent material subsequently absorbs the fluid from the wound contact material. The superior retention properties of the absorbent material mean it can retain the wound fluid and keep it away from the wound bed. Beneficially, this can enhance the healing rate of the wound. As described, when the absorbent layer is not punched into the wicking layer, the absorption properties and rate of fluid uptake are reduced.

The wound dressing of the present invention maintains a quick fluid uptake rate when the wound dressing is exposed to fluid, such as wound exudate. Thus, the wound dressing is beneficial in that wound exudate can be absorbed from the wound bed reducing the potential for wound breakdown or maceration.

The term 'wound' is used herein to refer to any breach or opening in the skin or subcutaneous tissue at a physiological target site of a human or animal. Typically, the present invention relates to a physiological target site of a human. The term physiological target site may also be referred to herein as a wound site.

The term 'wound dressing' is used herein to refer to materials placed on a wound at a wound site that have absorbent, gelling, adhesive or protective properties. The wound dressings are not limited to a particular size or shape. The wound dressings may be placed in direct or indirect contact with the wound.

The term 'water-swellable' is used herein to refer to a material that, when contacted with water or water-containing fluid, will absorb the fluid and swell, but will not substantially dissolve in that fluid. In some instances, the material will gel upon contact with water or a water-containing fluid.

The term 'water soluble' is used herein to refer to a material that, when contacted with water or a water-containing fluid, will readily dissolve in that fluid.

The wicking layer material comprises a foam, such as a polymeric foam material, that is not an absorbent or superabsorbent material. The polymeric foam may be polyurethane foam or polyethylene foam.

The absorbent or superabsorbent material may comprise a material selected from, for example, starch, cellulose and polymeric materials such as poly(vinyl alcohol) (PVA), poly(ethylene oxide) (PEO), and poly(acrylic acid). The poly(acrylic acid) may be a partially neutralised, lightly cross-linked poly(acrylic acid).

The absorbent material may be chemically modified. For example, the absorbent material may be a polymeric material obtained by graft polymerisation of acrylic acid onto the chain of carboxymethyl cellulose.

The terms "cross-linking" or "cross-linked" are used herein to refer to two or more polymer chains being linked by a primary bond, such as a covalent bond.

The term "lightly cross-linked" is used herein to refer to embodiments wherein the number of cross-linking primary bonds in the superabsorbent material is less than the total number of possible cross-linking bonds.

In some embodiments, the absorbent material is selected from, but not limited to, polymeric materials such as PVA, PEO, and poly(acrylic acid), preferably a partially neutralised, lightly cross-linked poly(acrylic acid).

Alternative absorbent materials include, but are not limited to, carboxymethylcellulose and chitosan fibre derivatives. For example, the chitosan fibre derivatives may comprise the materials described in WO 2010/031995. Thus, the absorbent material may comprise a blend of chitosan fibres with a material that has an acid associated therewith, e.g. cellulose fibre coated with an acid such as acetic and/or lactic acid.

Typically, the absorbent material is a partially neutralised, lightly cross-linked poly(acrylic acid).

The absorbent material is in the form of fibres. The fibres may be up to about 100 mm in length, preferably from about 5 to about 75 mm, more preferably from about 10 to about 60 mm and most preferably from about 30 to about 55 mm. Good results have been observed using fibres in the range of about 38 to about 52 mm in length.

The absorbent material is in the form of a non-woven fibrous layer. Where the absorbent material is in the form of a layer, it may comprise a wound facing surface and non-wound facing surface.

The term "wound facing surface" is used herein to refer to a surface of a layer of material that, in use, faces toward the wound site. The term "non-wound facing surface" refers to a surface of a layer of material that, in use, faces away from the wound site.

The absorbent material may gel on contact with water or body fluid(s). The absorbent material may be a gelling or semi-gelling material.

The term 'gelling material' is used herein to refer to a material in which substantially all of the components therein may gel upon contact with water or body fluid(s). For example, it may comprise a fibrous material wherein substantially all of the fibres are capable of gelling upon contact with water or body fluid(s).

The term 'semi-gelling' is used herein to refer to a material that comprises a mixture of components, some of which gel upon contact with water or body fluid(s) and some of which do not. For example, a semi-gelling absorbent material may comprise a combination of fibres, some of which gel upon contact with water or body fluid(s) and some of which do not.

The term 'non-gelling' is used herein to refer to a material in which substantially all of the components therein do not gel upon contact with water or body fluid(s). For example, it may comprise a fibrous material wherein substantially all of the fibres are incapable of gelling upon contact with water or body fluid(s).

The layer of wicking material is attached to the layer of absorbent material by an adhesive, such as heat-bonding, a pressure sensitive adhesive or heat meltable adhesives.. The adhesive material may consist of, or may comprise, any suitable physiologically acceptable adhesive. The adhesive bonding layer may attach the two materials by heat bonding or pressure bonding. Preferably, heat bonding is used. The adhesive bonding layer is preferably in powder form. The adhesive bonding layer may comprise, or consist of, a polymeric material selected from polycaprolactone, polyamides, polyesters, ethylene copolymers and combinations of any two or more thereof. Further, a superabsorbent polymer material may be used between these two layers, to aid in the absorption and fluid handling properties, maintaining moisture to prevent the absorbent layer drying out when the dressing uses a highly breathable outer layer.

According to one embodiment of the invention, there may be a wound contact adhesive layer.

The adhesive material may consist of, or may comprise, any suitable physiologically acceptable adhesive.

The adhesive material may be a pressure sensitive adhesive, a heat-bonding adhesive, a silicone adhesive and the like. Typically, the adhesive material is a silicone adhesive. In some embodiments, the adhesive material may be attached to a carrier layer, with a second adhesive material on the side distal to the wound. In such cases, the proximal surface (wound contact) comprising a silicone, polyurethane or acrylic adhesive, whilst the distal surface to the wound has coated a pressure sensitive adhesive. This carrier layer is perforated to allow fluid transfer through to the wicking layer. In other embodiments, the wound contact adhesive is coated directly onto the wicking layer, such that passageways are available to allow the fluid to pass through this adhesive layer to the wicking layer.

The adhesive material may be polymeric. Typically, the adhesive material is selected from acrylic adhesives, polyurethane adhesives, and silicone adhesives.

The adhesive material may be in the form of a layer. The adhesive layer comprises a wound facing surface and a non-wound facing surface.

According to one embodiment of the invention, there may be an anchor layer connected to the non-wound facing surface of the layer of absorbent material. This anchor layer is typically connected using an adhesive material.

The adhesive material may cover the whole or a part of a non-wound facing surface of the anchor material. The adhesive material may cover around 50-100% of the non-wound facing surface of the anchor material, preferably from around 70-80% coverage and most preferably around 75% coverage. In some embodiments, the adhesive material may cover 100% of the non-wound facing surface of the anchor material. It is noted that, the higher the coverage of the adhesive material, the stronger the bond between the adhesive material and the anchor material.

Where the adhesive material covers less than 100% of the non-wound facing surface of the anchor material, it may be located in intervals across such a surface. In such embodiments, the adhesive material may be located in regular or irregular intervals, preferably regular intervals.

The adhesive material may have a surface area greater than that of the anchor material and the absorbent material. In such embodiments, the adhesive material provides a border that outwardly extends beyond one or more edges of the anchor material and the absorbent material. Beneficially, the adhesive material provides a dual purpose of (a) providing an area of adhesive material to adhere to the anchor material and (b) providing a border around the adhered anchor material which can adhere to the skin surrounding the wound site. The adherence of the adhesive material to the skin of a patient can hold the wound dressing composition in place during use.

Patent GB1239921 teaches the art of stitching a foam to a fabric such that there are stitch yarns on the surface of the foam remote from the fabric and in which the foam and the fabric are laminated by adhesive or flame bonding such that the abrasion resistance of the foam surface which is still exposed is enhanced.

In contrast, in this present invention, the nonwoven superabsorbent material is not stitched through the foam, as a stitch involves a loop of thread or yarn resulting from a single pass or movement. The fibres from the superabsorbent material are punched through the foam, creating a passageway of fibres, which may slightly protrude from the surface of the foam distal to the superabsorbent nonwoven, due to the lack of stitching of the nonwoven superabsorbent material, so there are no loops. Prior to this punching, the two layers, the wicking layer and absorbent layer are bonded together.

The wound dressing composition may be multi-layered. For example, the wound dressing may be in the form different layers, comprising a wound contact adhesive layer, a wicking material and an absorbent material layer. The multilayer wound dressing has been described herein as comprising first, second and third layers, although it may comprise further layers, such fourth, fifth, sixth, seventh, eighth, ninth, tenth layers, or more. The further layers may comprise any of the features referred to herein in relation to the first, second or third layers such as a blend of superabsorbent polymer and heat meltable particles bonded between the wicking layer and the absorbent material layer.

The wound dressing may additionally comprise further components of a wound dressing, such for example, a backing material.

The backing may comprise medical grade sheet materials such as but not limited to polymer films, thin foams and fabrics e.g. polyurethane films, polyurethane foams, nonwoven fabrics, etc.

Suitable skin contact adhesives may include, but are not limited to, acrylate, silicone, or polyurethane based adhesives. They can be based on hydrogels and can be porous to moisture with a high moisture vapour transmission rate. They can be applied from water emulsions, solvents or using hot melt systems. The adhesives should have a good skin tack but give minimal skin trauma on removal. They can constitute 100% coverage of the backing, or a partial coverage thereof in the form of a pattern or mesh.

The wicking material is preferably in the form of a foam. The wicking layer comprises a wound facing surface and a non-wound facing surface. In some embodiments, the foam layer may have a thickness of from about 0.5 mm to about 7 mm, preferably from about 1.5 mm to about 3.0 mm.

Typically, the wicking material is a hydrophilic material, such as a hydrophilic foam. The wound contact material may comprise, or consist of, a hydrophilic polymeric material. The term 'hydrophilic' is used herein to refer to a material that has an affinity with water. In the present invention, the hydrophilic wound contact material has an affinity for water, which enables it to readily absorb water containing wound fluid.

However, as described this layer may be hydrophobic. The term 'hydrophobic' is used herein to refer to a material that has a low affinity for water.

Typically, the wicking material comprises, or consists of, polyurethane or polyethylene. The polyurethane is in the form of a foam. The polyethylene is in the form of a foam.

Preferably, the wicking material comprises, or consists of, a polyurethane foam.

The wicking material may cover the whole or a part of the wound facing surface of the absorbent material. Typically, the wicking material covers the whole of the wound facing surface of the absorbent material, behind the wound contact adhesive layer. However, in certain situations there may be no wound contact adhesive layer.

The wound dressing composition may further comprise a backing material. The backing material is typically attached to the adhesive material. The backing material is typically in the form of a layer, and represents the outermost layer, or the layer furthest away from the skin, of the wound dressing composition.

Beneficially, the backing material can act as a barrier to prevent contamination of the wound by contaminants such as bacteria. The backing material may also be waterproof.

Typically, the backing material forms a layer. The backing layer may be attached to the adhesive material by any appropriate means known to a person skilled in the art. Where the adhesive material comprises, or consists of, a pressure sensitive adhesive, the backing material can simply be contacted with the adhesive material and appropriate pressure applied. The backing material is preferably in the form of a film, more preferably a non-woven film.

The backing material may comprise, or consist of, a polymeric material. Typically, the backing material comprises, or consists of, polyurethane, polyethylene, and polyester. The polyester may be non-woven. Preferably, the backing material is polyurethane.

The wound dressing may further comprise a skin protection layer.

The skin protection layer may provide an alternative means of adhering the wound dressing composition to the skin surrounding the wound site. In such embodiments, the skin protection layer may be attached to the wound facing surface of the adhesive material. Preferably, the skin protection layer is attached to the wound facing surface of the border of the adhesive material, *i.e.* the part of the adhesive material that extends outwardly from the anchor material adhered thereto.

The skin protection layer contains an adhesive material for securing the dressing to the wound site, which may consist of a silicone material or pressure sensitive adhesive material. Silicone is ideally suited to application as the skin protection layer, since it can adhere to the skin in the same way as the adhesive material can, but it can be removed and reapplied with little irritation and damage. Also, the silicone material can be removed from the skin with reduced pain for the user compared to the adhesive material described herein.

The silicone layer may require a carrier material located between it and the adhesive material. The carrier material typically comprises a polymeric film. The carrier material may be the same material as the backing layer as described herein.

The skin protection layer adhered to the adhesive material may overlap with the non-wound facing surface of the anchor material. In such embodiments, the non-wound facing surface of the anchor material is attached to the adhesive material, as described herein, and is also attached to a part of the skin protection layer. This provides an increased stability for the wound dressing composition.

Alternatively, the skin protection layer may overlap with at least a part of the wound facing surface of the absorbent material or, if present, the wound facing surface of the wound contact material. Again, this provides increased stability to the wound dressing. It also provides a section of the skin protection layer that could be in direct contact with the wound site. Again, the gentle adherence of silicone material to the skin makes it ideally suited for contact with the wound site. Wounds and parts of wounds heal at different rates and stick to the wound dressing composition in different places and at different times. It is considered to be beneficial to have at least a part of the absorbent material, or the wound contact material if present, covered with a silicone material due to its gentle adherence to the wound site.

In some embodiments, the skin protection layer can extend across all or a part of the wound facing surface of the absorbent material or the wound contact material. In such embodiments, the skin protection layer is preferably perforated to facilitate absorption of the wound fluid by the combination of the wicking material and the absorbent material whilst also providing a breathable composition and one that is gently adhered to the wound site.

The wound dressing of the present invention may also comprise additional components mixed with any one or more of the material or layers described herein. Such additional components include, but are not limited to, pharmaceutical agents; wetting agents such as surfactants; growth factors; cytokines; agents which absorb agents which delay healing such as MMP's (matrix metalloproteinases) and elastase, and/or another wound dressing component, such as calcium, vitamin K, fibrinogen, thrombin, factor VII, factor VIII, clays such as kaolin, oxidised regenerated cellulose, gelatin, or collagen, etc.

Typical levels of any of these additional components could be from about 50 ppm up to about 50% by weight of the wound dressing composition. More typical levels would be less than 10%, still more typically less than about 5% by weight of the wound dressing composition. Additional components comprising less than about 1% by weight of the wound dressing composition are also envisaged by the present invention.

According to a further aspect of the present invention, there is provided a method of manufacturing a wound dressing as described herein, comprising the steps of:
(a) providing a layer of a wicking material comprising a foam and a layer of an absorbent material which is a nonwoven superabsorbent material;
(b) attaching the layer of wicking material to the layer of absorbent material by an adhesive to form bonded layers; and
(c) punching a plurality of holes in the bonded layers.

Typically, the adhesive is a heat meltable adhesive. According to one embodiment of the invention, an amount of a heat meltable adhesive is applied either to the layer of wicking material or the layer of an absorbent material. More typically, the heat meltable adhesive is applied to the layer of absorbent material. In this embodiment, the layer of absorbent material is placed onto the upper side (i.e. non-wound face side) of the absorbent layer, and the combined layers are passed through a heat chamber, wherein the dry powder of adhesive melts and bonds the layers together.

According to another embodiment of the invention, the holes are punched in the combined layers using a roller with multiple layers of needles thereon, such that the now bonded materials pass between the roller and another surface. The bonded layers are oriented so that the needles are on the side of the layer of absorbent material, in order that they can punch the fibres of the absorbent material through the wicking layer.

The method of the present invention may also comprise attaching an anchor layer and a backing layer, or a wound contact adhesive layer, as defined herein above.

The material and/or components of the wound dressing described herein may be provided in a sterile or non-sterile form. Where the materials and/or components are initially provided in a sterile form, sterilisation may be carried out using any of the methods conventionally known in the art, such as gamma irradiation, electron beam treatment, heat treatment, x-ray, etc., or it may alternatively be carried out by treatment using ethylene oxide. Sterilisation using ethylene oxide is preferred. A material in a non-sterile form may be provided in combination with one or more preservatives. However, it is preferred that the wound dressing composition is provided in a pre-sterilised form.

The wound dressing of the present invention is typically sterilised prior to packaging using any of the methods described herein. This enables the physician or emergency responder to use the wound dressing directly from the packaging, thus saving time.

According to a further aspect of the present invention, there is provided a use of a wound dressing as defined herein, or a wound dressing as define herein, in absorbing fluid discharged from a physiological target, or in stemming a flow of a fluid discharged from a physiological target site.

According to a further aspect of the present invention, there is provided a wound dressing as defined herein, or a wound dressing as defined herein, for use in absorbing fluid discharged from a physiological target, or for use in stemming a flow of a fluid discharged from a physiological target site.

Embodiments of the present invention will now be further described with reference to the following non-limiting examples and accompanying figures in which:
- Figure 1:: is a cross-sectional representation of a wound dressing according to an embodiment of the present invention;
- Figure 2:: is a cross-sectional representation of an alternative wound dressing of the present invention;
- Figure 3:: is a cross-sectional representation of a wound dressing of the present invention comprising a skin protection layer;
- Figure 4:: is a cross-sectional representation of a further alternative wound dressing of the present invention comprising a skin protection layer;
- Figure 5:: is a cross-sectional representation of a further alternative wound dressing of the present invention comprising a skin protection layer;
- Figure 6:: is a cross-sectional representation of a further alternative wound dressing of the present invention,
- Figure 7:: is a cross-sectional representation of the wound dressing of Figure 6 further comprising a skin protection layer;

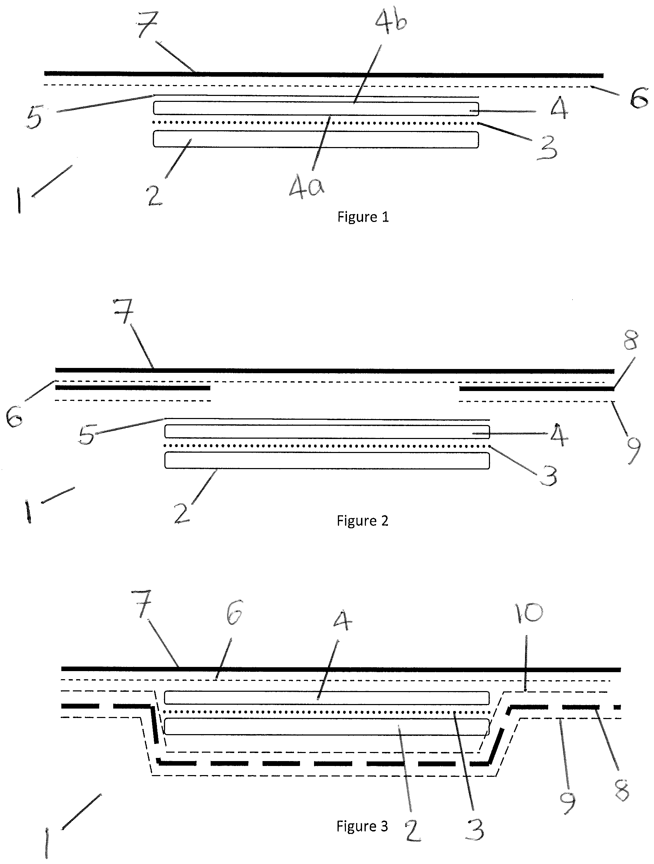

Referring to Figures 1 and 2, there is shown a wound dressing (1) comprising a layer of wicking material (2), a layer of meltable adhesive (3), a layer of absorbent material (4), layer of anchor material (5) a layer of adhesive material (6), and a backing layer (7). The layer of absorbent material (4) is punched into the layer of wicking material (2). The layer of wicking material (2) can act as a wound contact layer. Figure 2 also has two layers around the border comprising of a carrier layer (8) and a skin friendly adhesive layer (9).

The layer of wicking material (2) is adjacent to the wound site and will come into direct contact with the wound upon application of the wound dressing (1) to a wound. The layer of wicking material (2) is attached to the wound facing surface of the absorbent material (4) by any of the means described herein. Preferably, the layer of wicking material (2) is attached to the layer of absorbent material (4) using a powder adhesive. The layer of wicking material (2) also serves to prevent or reduce the leaching of fluids from the layer of absorbent material (4).

The meltable adhesive (3) is positioned between the layer of wicking material (2) and the absorbent material layer (4). In Figures 1 and 2, the bond created between these layers is such that it will not break when the respective materials get wet with wound fluid during use.

The anchor material (5) is attached to the non-wound facing surface of the absorbent material (4). Typically, the anchor material (5) is heat-bonded to the absorbent material (4). As described herein, the bond created between the anchor material (5) and the absorbent material (4) is such that it will not break when the respective materials get wet with wound fluid during use.

The adhesive layer (6) has a backing layer (7) attached to its non-wound facing surface. As with the anchor material (5), the backing layer (7) can be attached to the anchor material (5) by contacting the two materials together and applying pressure.

As can be seen in both Figures 1 and 2, the adhesive layer (6) and the backing layer (7) have a greater cross-sectional area than the anchor material (5), the absorbent material (4) and the layer of wicking material (2), creating a border portion (8). The wound facing surface of the backing layer (7) in the border portion (8) is, in use, applied directly to the patient's skin surrounding the wound site with the use of an adhesive (10). Thus, the adhesive layer (10) has the dual purpose of adhering to the anchor layer (5) and the skin of the patient.

Figure 3 is an alternative design, whereby a perforated layer, comprising of a carrier layer, a layer of a pressure sensitive adhesive proximal to the absorbent layer (4) and a silicone adhesive layer proximal to the wound is used to envelope the fluid absorbing portion of the dressing. In doing so there is no requirement for the anchor layer.

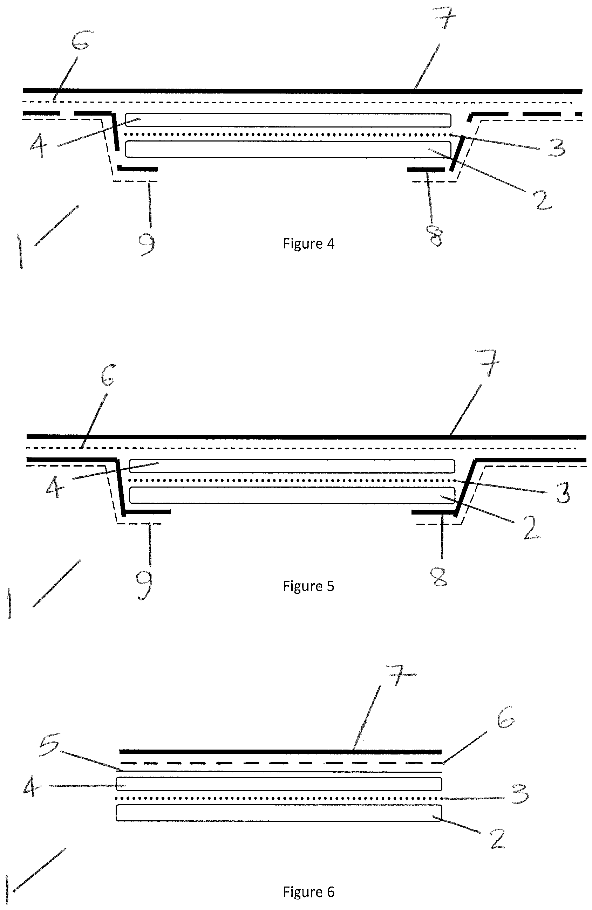

Figures 4 and 5 are alternatives to Figures 1 and 2 whereby the adhesive portion of the dressing that is used to secure to the persons skin/wound overlaps the wound contact layer, but not fully across this layer. This layer can be perforated or non-perforated.

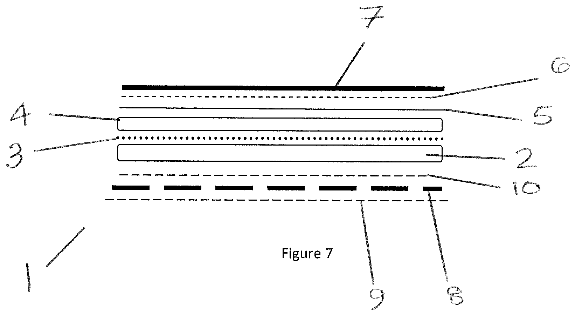

Figures 6 and 7 show a non-bordered version with and without an adhesive face.

In use, the wound dressing of the present invention is applied to a wound by contacting the wicking layer and/or the silicone layer with the wound site. The wound dressing can be affixed to the patient's skin by applying downward pressure to the border portion or the non-bordered part where no border is present, or by means of a secondary securement device. Wound exudates from the wound will be absorbed through the wicking layer and into the superabsorbent layers. This has the effect of drawing fluid away from the wound bed, creating a moisture level at the wound bed that is more conducive to healing.

### Examples

The wound dressing of the present invention do not delaminate, maintain a level of moisture and have a fast wicking rate under 60 seconds, preferably under 30 seconds for 1 ml of fluid. To test this, the following experiment was followed.

### Test Methodology

Two island wound dressings in conjunction with the present invention as shown in Figure 3 were prepared. The first wound dressing (T1) was made up of a superabsorbent layer of polyacrylate fibres gsm 200, and a pattern coated adhesive layer of a pressure-sensitive acrylic adhesive 20 gsm, a backing layer of a highly breathable polyurethane film, 30 micron thickness and an wicking layer of a polyurethane foam, 1.5 mm thickness and a superabsorbent polymer/dry hot meltable adhesive layer at 0.49g per 100 cm². The superabsorbent layer was bonded to the wicking layer and then punched into the wicking layer. The second wound dressing (T2) was made up of a superabsorbent layer of polyacrylate fibres gsm 250, and a pattern coated adhesive layer of a pressure-sensitive acrylic adhesive 20 gsm, a backing layer of a highly breathable polyurethane film, 30 micron thickness and an wicking layer of a polyurethane foam, 1.5 mm thickness and a dry hot meltable adhesive layer at 0.25g per 100 cm². The superabsorbent layer was bonded to the wicking layer and then punched into the wicking layer.

Two control dressings (C1 and C2) were constructed as described above, whereby the superabsorbent layers were not punched into the wicking layer.

All test dressings and control were packaged and sterilised using ethylene oxide sterilisation.

In the tests, Solution A is 142 mmol sodium ions and 2.5 mmol calcium ions as the chloride salt, Solution B is saline and Solution C is simulated wound fluid (50% peptone water and 50% fetal bovine serum).

To each test article and control, 1ml of each of the solutions A, B and C were pipetted onto the adhesive wound contact surface of the dressings, turned such that the adhesive wound contact surface was uppermost from the bench. This forms a droplet of solution on the surface of the dressing. The time taken for the solution to be absorbed into the dressing was recorded.

| Test article | Average time taken for 1ml solution to be absorbed into the test article (seconds) | | |
|---|---|---|---|
| | Solution A | Solution B | Solution C |
| T1 | 10 | 9 | 16 |
| T2 | 12 | 10 | 20 |
| C1 | 70 | 75 | 90 |
| C2 | 74 | 80 | 86 |

The above result show that the punching of the superabsorbent layer into the wicking layer significantly decreases the time taken for the fluid to be absorbed into the wound dressing.

It is of course to be understood that the present invention is not intended to be restricted to the foregoing examples which are described by way of example only.

## Claims

1. A wound dressing comprising a first layer of a wicking material and a second layer of an absorbent fibre material, whereby the absorbent material is punched into and/or through the wicking material,
wherein the wicking material comprises a foam, and the absorbent fibre material is a nonwoven superabsorbent material;
wherein at least a part of the absorbent material is exposed on a wound facing surface of the wicking layer; and
wherein prior to punching the absorbent material through the wicking material, the wicking material and the absorbent fibre material are bonded together by an adhesive to form bonded layers.

2. A wound dressing according to claim 1, wherein the absorbent material comprises a polymeric material.

3. A wound dressing according to any preceding claim, wherein the polymeric material is selected from PVA, PEO and polyacrylic acid.

4. A wound dressing according to any preceding claim, wherein the fibres form a non-woven layer.

5. A wound dressing according to any preceding claim, wherein the wicking material comprises either a hydrophilic or hydrophobic material.

6. A wound dressing according to claim 5, wherein the hydrophilic material is selected from polyurethane foams, and the hydrophobic material is selected from polyethylene foams.

7. A method of manufacturing a wound dressing according to any of claims 1-6, comprising the steps of:
(a) providing a layer of a wicking material comprising a foam and a layer of an absorbent material which is a nonwoven superabsorbent material;
(b) attaching the layer of wicking material to the layer of absorbent material by an adhesive to form bonded layers; and
(c) punching a plurality of holes in the bonded layers.

8. A method according to claim 7, wherein the layers are bonded using a heat meltable adhesive.

9. A method according to claim 7 or claim 8, wherein the plurality of holes is punched in the bonded layers using a roller having a plurality of needles thereon.

## Patentansprüche

1. Wundverband, bestehend aus einer ersten Schicht eines feuchtigkeitstransportierenden Materials und einer zweiten Schicht eines saugfähigen Fasermaterials, wobei das saugfähige Material in und/oder durch das feuchtigkeitstransportierende Material gestanzt ist,
wobei das feuchtigkeitstransportierende Material einen Schaumstoff aufweist und das saugfähige Fasermaterial ein super-saugfähiges Vliesstoffmaterial ist;
wobei mindestens ein Teil des saugfähigen Materials auf einer wundzugewandten Oberfläche der feuchtigkeitstransportierenden Schicht freiliegend ist; und
wobei vor dem Durchstanzen des saugfähigen Materials durch das feuchtigkeitstransportierende Material das feuchtigkeitstransportierende Material und das saugfähige Fasermaterial durch einen Klebstoff miteinander verbunden werden, um verbundene Schichten zu bilden.

2. Wundverband nach Anspruch 1, wobei das saugfähige Material ein Polymermaterial aufweist.

3. Wundverband nach einem der vorhergehenden Ansprüche, wobei das Polymermaterial aus PVA, PEO und Polyacrylsäure ausgewählt wird.

4. Wundverband nach einem der vorhergehenden Ansprüche, wobei die Fasern eine Vliesstoffschicht bilden.

5. Wundverband nach einem der vorhergehenden Ansprüche, wobei das feuchtigkeitstransportierende Material entweder ein hydrophiles oder hydrophobes Material aufweist.

6. Wundverband nach Anspruch 5, wobei das hydrophile Material aus PolyurethanSchäumen ausgewählt wird und das hydrophobe Material aus Polyethylen-Schäumen ausgewählt wird.

7. Verfahren zum Herstellen eines Wundverbands nach einem der Ansprüche 1 bis 6, der folgenden Schritte aufweist:
(a) Bereitstellen einer Schicht aus feuchtigkeitstransportierendem Material, bestehend aus einem Schaumstoff, und einer Schicht eines saugfähigen Materials, welches ein super-saugfähiger Vliesstoff ist;
(b) Befestigen der Schicht des feuchtigkeitstransportierenden Materials an der Schicht des saugfähigen Materials mittels eines Klebstoffs zum Bilden verbundener Schichten; und
(c) Stanzen mehrerer Löcher in die verbundenen Schichten.

8. Verfahren nach Anspruch 7, wobei die Schichten mittels eines wärmeschmelzbaren Klebstoffs verbunden werden.

9. Verfahren nach Anspruch 7 oder 8, wobei die mehreren Löcher in die verbundenen Schichten mittels einer Walze gestanzt werden, die mehrere Nadeln darauf aufweist.

## Revendications

1. Pansement comprenant une première couche d'un matériau à effet mèche et une deuxième couche d'un matériau fibreux absorbant, moyennant quoi le matériau absorbant est percé dans et/ou à travers le matériau à effet mèche,
dans lequel le matériau à effet mèche comprend une mousse, et le matériau fibreux absorbant est un matériau superabsorbant non tissé ;
dans lequel au moins une partie du matériau absorbant est exposée sur une surface de la couche à effet mèche faisant face à la plaie ; et
dans lequel, avant le perçage du matériau absorbant à travers le matériau à effet mèche, le matériau à effet mèche et le matériau fibreux absorbant sont liés l'un à l'autre par un adhésif pour former des couches liées.

2. Pansement selon la revendication 1, dans lequel le matériau absorbant comprend un matériau polymère.

3. Pansement selon une quelconque revendication précédente, dans lequel le matériau polymère est sélectionné parmi PVA, PEO et acide polyacrylique.

4. Pansement selon une quelconque revendication précédente, dans lequel les fibres forment une couche non tissée.

5. Pansement selon une quelconque revendication précédente, dans lequel le matériau à effet mèche comprend un matériau hydrophile ou hydrophobe.

6. Pansement selon la revendication 5, dans lequel le matériau hydrophile est sélectionné parmi des mousses de polyuréthane, et le matériau hydrophobe est sélectionné parmi des mousses de polyéthylène.

7. Procédé de fabrication d'un pansement selon l'une quelconque des revendications 1 à 6, comprenant les étapes consistant à :
(a) fournir une couche d'un matériau à effet mèche comprenant une mousse et une couche d'un matériau absorbant qui est un matériau superabsorbant non tissé ;
(b) attacher la couche de matériau à effet mèche à la couche de matériau absorbant par un adhésif pour former des couches liées ; et
(c) percer une pluralité de trous dans les couches liées.

8. Procédé selon la revendication 7, dans lequel les couches sont liées à l'aide d'un adhésif thermofusible.

9. Procédé selon la revendication 7 ou la revendication 8, dans lequel la pluralité de trous sont percés dans les couches liées en utilisant un rouleau présentant une pluralité d'aiguilles sur celui-ci.
